(19)

Europäisches
Patentamt
European
Patent Office
Office européen
des brevets

(11) **EP 4 613 190 A1**

(12) **EUROPEAN PATENT APPLICATION**

(43) Date of publication:
**10.09.2025 Bulletin 2025/37**

(21) Application number: **25159445.3**

(22) Date of filing: **21.02.2025**

(51) International Patent Classification (IPC):
**A61B 5/03** (2006.01)       **A61B 5/07** (2006.01)
**A61B 5/20** (2006.01)       **A61B 5/00** (2006.01)

(52) Cooperative Patent Classification (CPC):
**A61B 5/036; A61B 5/073; A61B 5/205;**
**A61B 5/4238; A61B 5/4255; A61B 5/746;**
A61B 5/037; A61B 2560/063; A61B 2562/0247

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **04.03.2024   US 202463627589 P**

(71) Applicant: **Dotspace Inc.**
**Wilmington, DE 19801 (US)**

(72) Inventors:
• **CHOU, Chao-Hsin**
  **Wilmington, 19801**
  **County of New Castle, Delaware (US)**
• **JOW, Ueiming**
  **Wilmington, 19801**
  **County of New Castle, Delaware (US)**

(74) Representative: **Rapisardi, Mariacristina**
**Ufficio Brevetti Rapisardi S.r.l.**
**Via Serbelloni, 12**
**20122 Milano (IT)**

(54) **METHOD AND SYSTEM FOR INTRA-ABDOMINAL PRESSURE EVALUATION AND WARNING**

(57)       A method for intra-abdominal pressure evaluation and warning is provided. The method includes: arranging a sensing device at an arrangement space inside an abdominal cavity of an object; measuring a pressure value of the arrangement space by a pressure sensing module of the sensing device; receiving the pressure value and recording pressure-time information by a control device; accumulating an area under a curve of the pressure-time information during a measuring duration; and, while the accumulated area exceeds a warning threshold, outputting a warning signal by the control device.

FIG. 1

EP 4 613 190 A1

## Description

## TECHNICAL FIELD

The present invention relates to a method and a system for intra-abdominal pressure evaluation and warning; in particular, the present invention relates a method and a system for measuring and evaluating the intra-abdominal pressure in real-time to provide a warning signal.

## BACKGROUND

In a clinical practice, for some patients, it is necessary to periodically monitor the intra-abdominal pressure (IAP) to avoid the occurrence of various complications that may endanger the patient's life. For example, patients may experience a continuous increasement of IAP, which leads to intra-abdominal hypertension (IAH) due to various clinical reasons. A prolonged and persistent IAH may lead to abdominal compartment syndrome (ACS), which causes significant harm to the patient's respiratory, cardiovascular, renal, gastrointestinal and the central nervous systems. Severe organ damage will cause symptoms with high mortality such as organ failure.

Conventional methods, such as measuring the bladder pressure by pumping water or air, measuring the variation of the abdominal circumference of the patient, or measuring the pressure from outside the patient's abdomen, can be used to derive the IAP of the patient. However, the method for measuring the bladder pressure makes the patient feel discomfort and causes a psychological or physiological stress to the patient. In addition, a long-term bladder stressing may lead to urinary system infections. Therefore, deriving IAP by measuring the bladder pressure cannot be implemented frequently or continuously to the patient. On the other hand, the accuracy is insufficient when estimating IAP through methods such as measuring abdominal circumference or measuring the pressure from the outside of the abdomen of the patient. Moreover, the mobility of the patient is significantly affected during measuring the abdominal circumference and measuring the pressure from outside the patient's abdomen. Accordingly, there is a need for continuous, long-term and real-time measurement method for monitoring patient's IAP. Moreover, the conventional IAP measurement methods should be performed by professional operators or implemented by specialized equipment. Therefore, the conventional IAP measurement methods are not easy for point-of-care applications or home care applications or other remote medical applications.

Because the conventional measurement methods for measuring IAP cannot provide continuous and real-time information, insufficient information of IAP leads to underestimation or misestimation of the level of IAH and delays the timing for IAH treatment. On the other hand, the insufficient information of IAP derived from discrete measurements usually leads to the level of IAP to be overestimated or underestimated. The overestimated or underestimated IAP results in misjudgment to the level of IAH and waste in medical resources due to over treatment or delay treatment. For example, the prolonged and intensified IAH will develop to ACS. If a patient experiences a sudden increasing IAP but subsequently alleviated, the risk of ACS is not so high that needs radical treatment. The low-risk situation can be treated by observing or providing simple/supportive treatments without excessive medical treatments. In other words, the level of IAH and the appropriate medical treatments for a patient suffering from IAH cannot be effectively determined or confirmed by a discrete measurement and/or a short-term measurement for IAP.

Accordingly, there are unmet needs to be overcome in the conventional methods of measuring IAP.

## SUMMARY

Therefore, the present invention provides a method and a system for intra-abdominal pressure evaluation and warning to effectively overcome the problems/issues encountered by the prior arts.

More specifically, one of the aspects of the present invention is to provide a method and a system for measuring IAP in a continuous, long-term and real-time manner. One of the aspects of the present invention is to provide a method and a system for measuring IAP that reduces the risk of underestimating, overestimating, or misestimating the level of IAH. One of the aspects of the present invention is to provide a method and a system for warning an abnormal level of IAP to prevent waste in human resources or medical resources.

The present invention provides a method for intra-abdominal pressure evaluation and warning. The method includes: arranging a sensing device at an arrangement space inside an abdominal cavity of an object; measuring a pressure value of the arrangement space by a pressure sensing module of the sensing device; receiving the pressure value and recording pressure-time information by a control device; accumulating an area under a curve of the pressure-time information during a measuring duration; and, while the accumulated area exceeds a warning threshold, outputting a warning signal by the control device .

In an embodiment, the Y-axis of the pressure-time information is represented as a ratio of the pressure value to a critical pressure value.

In an embodiment, the sensing device is arranged at the arrangement space via an esophagus of the object.

In an embodiment, the arrangement space is selected from any location in a digestive system of the object.

In an embodiment, the sensing device is communicatively coupled to the control device via a wireless communication.

In an embodiment, the pressure sensing module includes a sensor with a piezoelectric, a piezoresistive, a capacitive, an inductive, an electromagnetic piezoelectric, or a thin-film pressure sensing mechanism.

The present invention provides a system for intra-abdominal pressure evaluation and warning. The system includes a sensing device and a control device. The sensing device is arranged at an arrangement space inside an abdominal cavity of an object. The sensing device includes a pressure sensing module configured to measure a pressure value of the arrangement space over time. The control device is coupled to the sensing device. The control device is configured to receive the pressure value and record pressure-time information. Wherein the control device is further configured to accumulate an area under a curve of the pressure-time information during a measuring duration, and output a warning signal while the area exceeds a warning threshold.

In an embodiment, the Y-axis of the pressure-time information is represented as a ratio of the pressure value to a critical pressure value.

In an embodiment, the sensing device is arranged at the arrangement space via an esophagus of the object.

In an embodiment, the arrangement space is selected from any location in a digestive system of the object.

In an embodiment, the sensing device further includes a wireless transmission module configured to establish a wireless communication; the control device is communicatively coupled to the sensing device via a wireless communication.

In an embodiment, the pressure sensing module includes a sensor with a piezoelectric, a piezoresistive, a capacitive, an inductive, an electromagnetic piezoelectric, or a thin-film pressure sensing mechanism.

Compared to the prior arts, the present invention measures IAP through a sensing device arranged inside an abdominal cavity of an object, and records IAP through a control device. The sensing device is able to measure IAP long-termly and continuously in the abdominal cavity of the object. The control device is configured to establish pressure-time information based on the recorded IAP with time stamps. The area under the curve of the pressure-time information can be used to evaluate the status of IAP to the object. For example, when the level of IAP is increasing and does not relief for a long time, the area under the curve will be larger than a normal level of IAP. In addition, the present invention also excludes occasional or sudden increasement of IAP. For example, when the level of IAP of the object can be relieved after a suddenly increasement, the area under the curve will also be lower than a warning threshold. Accordingly, the present invention reduces the risk of underestimating, overestimating, or misestimating the level of IAH, or avoids waste of the human resources and the medical resources by calculating the area under the curve of the pressure-time information of IAP.

On the other hands, compared to the prior arts, the present invention transmits or receives wirelessly through a wireless sensing device having a transmission module. The wireless sensing device is arranged inside a location of the digestive system of the object, such as the stomach or intestines, via the esophagus of the object. Preferably, the wireless sensing device is a capsule sensor. The wireless sensing device provides information of IAP to the control device through a wireless transmission; therefore, the mobility of the object will not be restricted by catheters or cables, which improves comfort to the object. In addition, the process of taking sedative or anesthetic drugs, which is used for calming down the object and avoiding interference during the measurement, can be avoided. Accordingly, the present invention achieves the purpose of continuous, long-term and real-time measurement. In addition, the present invention is also beneficial for the applications such as point-of-care or home cares by wireless transmission.

## BRIEF DESCRIPTION OF THE DRAWINGS

The accompanying drawings are presented to help describe various aspects of the present invention. In order to simplify the accompanying drawings and highlight the contents to be presented in the accompanying drawings, conventional structures or elements in the accompanying drawings may be drawn in a simple schematic way or may be omitted. For example, a number of elements may be singular or plural. These accompanying drawings are provided merely to explain these aspects and not to limit them.

FIG. 1 illustrates the flowchart of the method for intra-abdominal pressure evaluation and warning according to an embodiment of the present invention.
FIG. 2 illustrates a block diagram of the system for intra-abdominal pressure evaluation and warning according to an embodiment of the present invention.
FIG. 3A illustrates a schematic diagram of the implementation for arranging a sensing device without incision according to an embodiment of the present invention.
FIG. 3B illustrates a schematic diagram of the implementation for invasively arranging a sensing device according to an embodiment of the present invention.
FIG. 4 illustrates a block diagram of the sensing device having a wireless transmission module according to an embodiment of the present invention.
FIG. 5 illustrates a pressure versus time graph according to an embodiment of the present invention.
FIG. 6 illustrates an abnormal pressure ratio versus time graph according to an embodiment of the present invention.

FIG. 7 illustrates a schematic diagram of the area under the curve of the pressure-time information according to an embodiment of the present invention.

FIG. 8 illustrates a schematic diagram of the area under the curve at the portion of the curve that exceeds the critical pressure value according to an embodiment of the present invention.

## DETAILED DESCRIPTION

Any reference to elements using terms such as "first" and "second" herein generally does not limit the number or order of these elements. Conversely, these names are used herein as a convenient way to distinguish two or more elements or element instances. Therefore, it should be understood that the terms "first" and "second" in the request item do not necessarily correspond to the same names in the written description. Furthermore, it should be understood that references to the first element and the second element do not indicate that only two elements can be used or that the first element needs to precede the second element. Open terms such as "include", "comprise", "have", "contain", and the like used herein means including but not limit to.

The term "coupled" is used herein to refer to direct or indirect electrical coupling between two structures. For example, in an example of indirect electrical coupling, one structure may be coupled with another structure through a passive element such as a resistor, a capacitor, or an inductor.

In the present invention, the term such as "exemplary" or "for example" is used to represent "giving an example, instance, or description". Any implementation or aspect described herein as "exemplary" or "for example" is not necessarily to be construed as preferred or advantageous over other aspects of the present invention. The terms "about" and "approximately" as used herein with respect to a specified value or characteristic are intended to represent within a value (for example, 10%) of the specified value or characteristic.

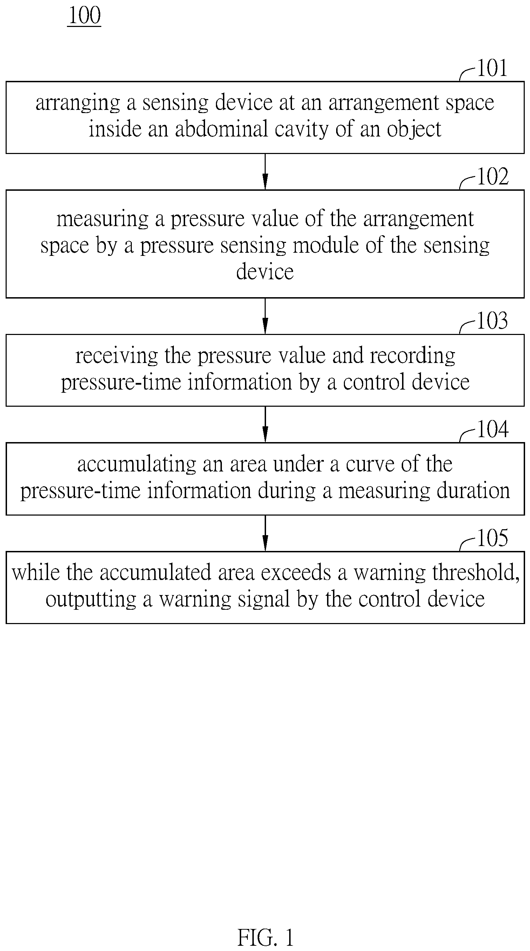

The present invention provides a method for intra-abdominal pressure (IAP) evaluation and warning. Refer to FIG. 1; FIG. 1 illustrates a flowchart of the method 100 for intra-abdominal pressure evaluation and warning. The method 100 includes arranging a sensing device at an arrangement space inside an abdominal cavity of an object (step 101); measuring a pressure value of the arrangement space by a pressure sensing module of the sensing device (step 102); receiving the pressure value and recording pressure-time information by a control device (step 103); accumulating an area under a curve of the pressure-time information during a measuring duration (step 104); and, while the accumulated area exceeds a warning threshold, outputting a warning signal by the control device (step 105).

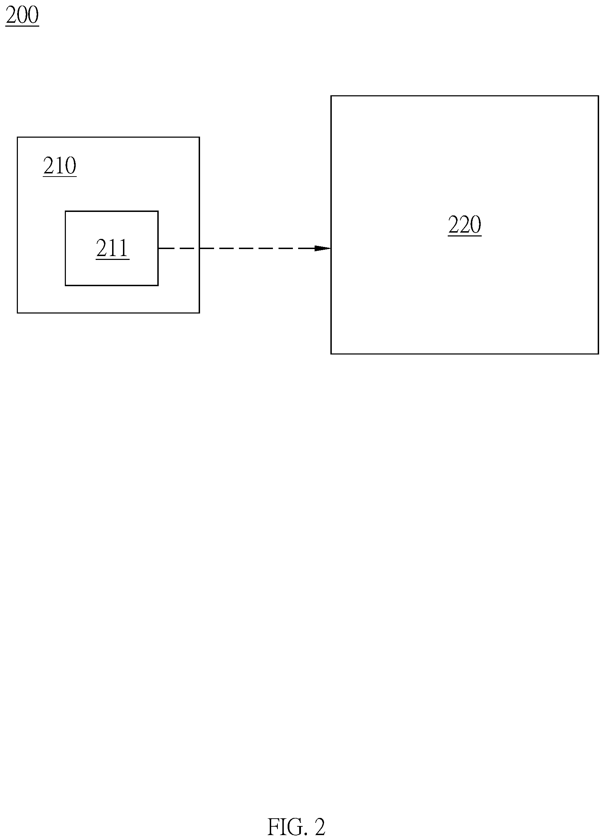

FIG. 2 illustrates a schematic diagram of an exemplary system 200 for implementing the method 100 of intra-abdominal pressure evaluation and warning. The system 200 for intra-abdominal pressure evaluation and warning includes a sensing device 210 arranged at an arrangement space inside an abdominal cavity of an object, and a control device 220 coupled with the sensing device 210. The sensing device 210 includes a pressure sensing module 211 configured to measure pressure values of the arrangement space. The control device 220 receives the pressure values from the sensing device 210 and records pressure-time information. The control device 220 is further configured to accumulate an area under a curve of the pressure-time information during a measuring duration, and output a warning signal while the area exceeds a warning threshold. It should be noted that the system 200 is an implementing example, and implementing means of the method 100 is not limited by the system 200.

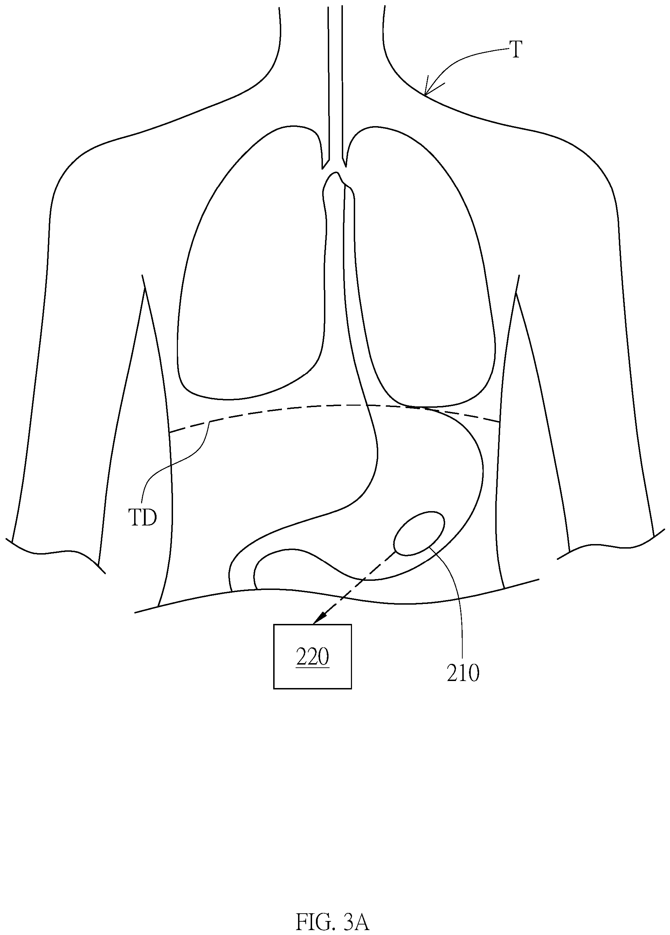

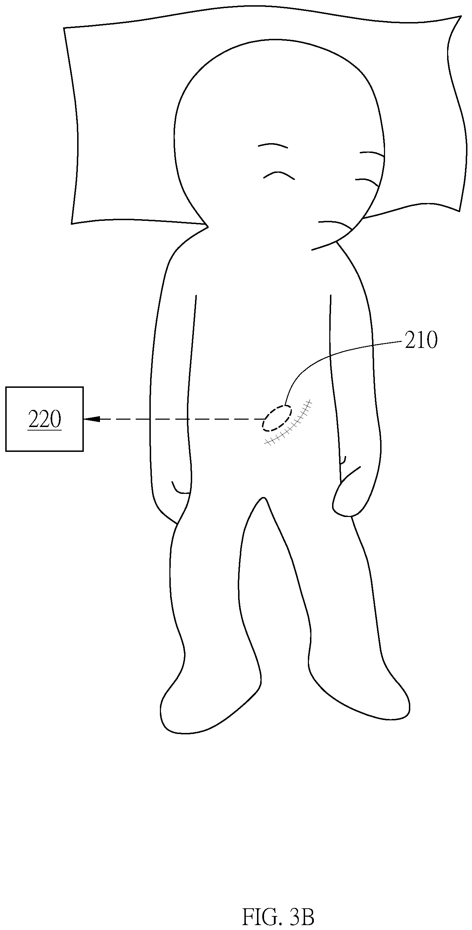

In the step 101 for arranging the sensing device 210, please refer to FIG. 3A. The sensing device 210 can be arranged in the abdominal cavity of the object (T) via the esophagus, rectum, vagina, or other suitable pathways. The incisionless approach can reduce the discomfort of the object (T) and can be applied for a long-term measurement. On the other hand, referring to FIG. 3B, the sensing device 210 can be invasively arranged in the abdominal cavity by creating an incision through the skin of the abdominal cavity of the object (T). By the invasive approach, the sensing device 210 can be positioned at an optimal location for IAP measurement to obtain accurate and immediate response to the IAP variation. It should be noted that in the present invention, the abdominal cavity can be exemplarily defined as a location between the diaphragm (TD) and the edge of the pelvic entrance, but not limited thereto. The abdominal cavity can be any location where the IAP can be properly measured. The arrangement space for the sensing device 210 can be referred to any suitable space in the digestive system such as stomach, intestines, or abdominal cavity.

In the step 102 for measuring the pressure value of the arrangement space, the pressure sensing module 211 of the sensing device 210 can be used to perform the measurement. The pressure sensing module 211 can be a sensor with a piezoelectric, a piezoresistive, a capacitive, an inductive, an electromagnetic piezoelectric, or a thin-film pressure sensing mechanism, but not limited thereto. The pressure sensing module 211 is configured to measure the pressure in the arrangement space (e.g. stomach). Because the pressure of the organs in the human body and the IAP can be approximated or extrapolated, the pressure of the arrangement space can be considered as the IAP.

In the step 103, the control device 220 is, for example, a consumer electronic device such as a computer, a smartphone, or a tablet, or an existing medical device, or a wire or wireless receiving device which is specifically designed for the sensing device 210. In another aspect, the control device 220 can be a circuit device composed of integrated circuits (ICs) with computing capabilities, such as FPGA (field programmable gate array), ASIC (application specific integrated circuit), microprocessors, and other components. The control device 220 is configured to receive the pressure values through a wired or wireless transmission. The control device 220 can record the pressure-time information based on the pressure values transmitted from the sensing device 210 over time in physical storage components (such as hard drives, memory, or optical discs) or a cloud storage, but not limit thereto.

In the implementation of wired transmission, the control device 220 and the sensing device 210 can be connected through catheters, cables and/or transmission lines. More specifically, when the sensing device 210 is located in the arrangement space (e.g. stomach), the sensing device 210 is physically connected to the control device 220 through a catheter and/or electrically coupled to the control device 220 through a transmission line or a cable. The catheters, cables and/or transmission lines may extend into the arrangement space via the esophagus of the object (T). In this way, the sensing device 210 is connected with the control device 220, and the power or signal can be directly provided via physical channels, which reduces energy loss or signal interference. Accordingly, the wired transmission improves the reliability and power persistence of the sensing device 210.

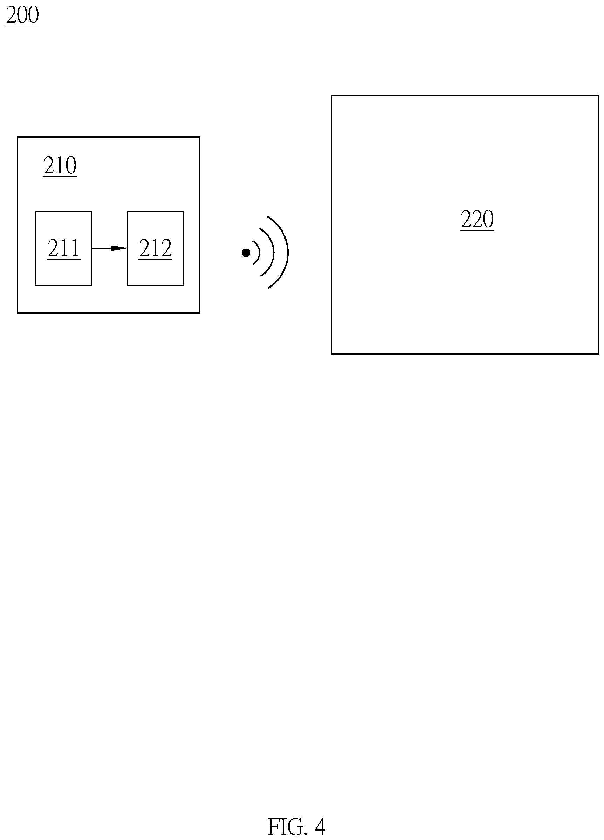

FIG. 4 illustrates an exemplary implementation of coupling the control device 220 with the sensing device 210 through a wireless transmission. In an embodiment, the sensing device 210 further includes a wireless transmission module 212, which is configured to establish a wireless transmission. The control device 220 can be communicatively coupled to the sensing device 210 through the wireless communication. In the embodiment, the sensing device 210 can be a capsule-shaped sensing device 210 that can be swallowed into the stomach via the esophagus, but not limited thereto. The sensing device 210 can be coupled to the control device 220 through conventional wireless communication methods such as Bluetooth, ZigBee, or Wi-Fi, but not limited thereto. The sensing device 210 provides pressure information to the control device 220 through the wireless transmission created by the wireless transmission module 212, which allows less restricted movements of the object (T) and reduces the discomfort of the object (T) when the catheter or cables is arranged. Therefore, the wireless transmission module 212 prevents the object (T) from taking sedative or anesthetic drugs or reduces the dosage of the sedative or anesthetic drugs that may interfere with the measurement. Accordingly, the present invention can achieve the purpose of continuous, long-term and real-time measurement. In addition, through the wireless transmission feature, the present invention can be beneficial for the applications such as point-of-care or home cares.

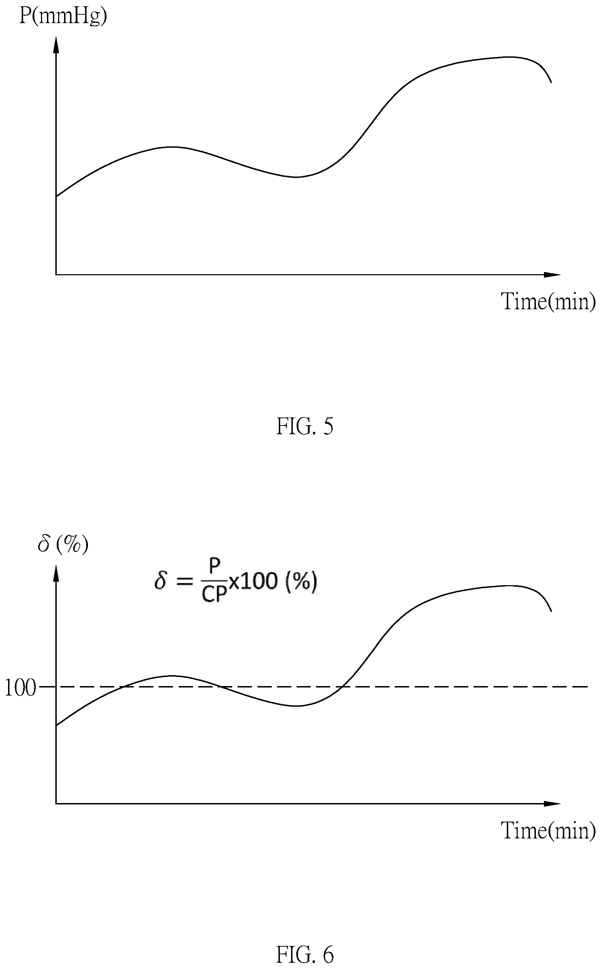

In the step 103 for recording and establishing the pressure-time information, for example, the sensing device 210 provides the measured pressure value to the control device 220, and the control device 220 can record and assign a timestamp to the pressure value received from the sensing device 210, so the pressure-time information can be established, but not limited thereto. The timestamp can be assigned/recorded synchronously while measuring the pressure value by the sensing device 210. The recorded pressure values with corresponding timestamp can be used to establish a pressure versus time (pressure-time) graph, such as the pressure-time curve shown in FIG. 5. In the pressure-time graph, the X-axis represents time, and the Y-axis represents the pressure value (P). In another aspect, the Y-axis can represent the ratio ($\delta$) of the pressure value (P) to a critical pressure value (CP). Specifically, referring to FIG. 6, the pressure value (P) can be divided by the critical pressure value (CP) to obtain the ratio ($\delta$), so the abnormal pressure ratio versus time graph can be derived, such as the abnormal pressure ratio-time curve shown in FIG. 6. The abnormal pressure ratio may be derived by the following equation:

$$\delta = \frac{P}{CP} \times 100\ (\%)$$

Wherein the critical pressure value (CP) can be the critical judgment value between a normal IAP and intra-abdominal hypertension (IAH) or can be set based on the medical experiences. For example, in an embodiment, the critical pressure value (CP) can be 12mmHg, but not limited thereto. Although the X-axis shown in FIGs. 5 to 6 is represented as time, in other embodiments, the X-axis can be represented as the number of data transactions or any time related parameters. The number of data transactions can be converted into time by considering the sampling rate of the sensing device 210. Moreover, the pressure-time relationship graphs shown in FIGs. 5 to 6 are only intended to illustrate the present invention and are not intended to limit the format that the control device 220 stores the pressure-time information.

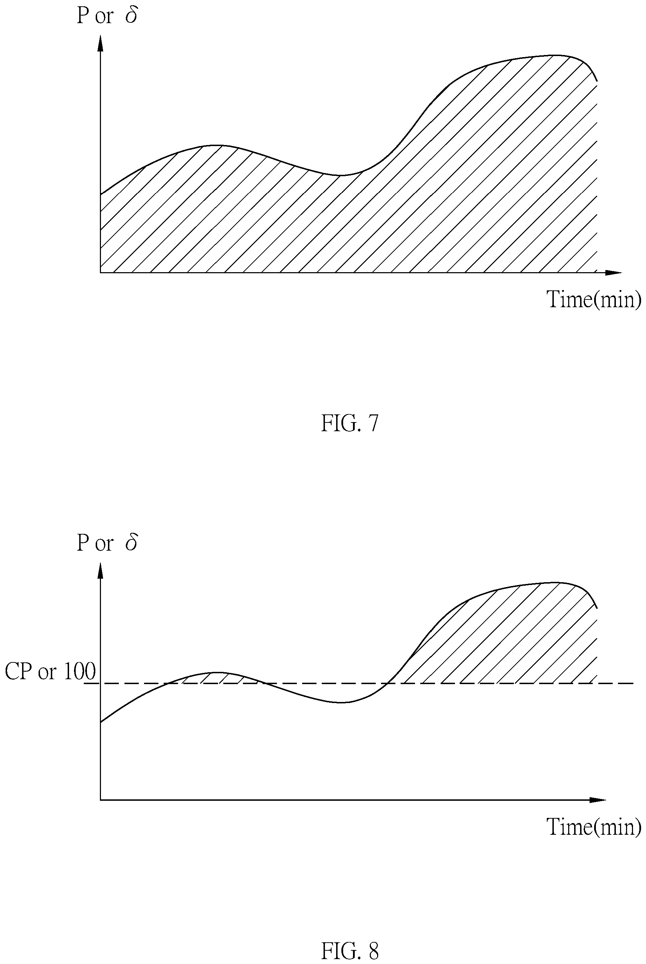

In step 104, during the measuring duration, the control device 220 calculates the area under the curve (AUC) of the pressure-time information. Specifically, the measuring duration can be defined by a preset duration or a continuous monitoring time period. The continuous monitoring time period can be a time slot from starting the monitoring process until the monitoring process is stopped, or the sensing device 210 is removed or released from the object. Referring to FIG. 7, the control device 220 accumulates the current area under the curve (e.g. current AUC) by conventional calculations such as integration of the pressure value (P) over the time. In an embodiment, the AUC can be approximately derived by adding the pressure values (P) measured per unit time, or performing integration operations on the pressure value as a function of time, but not limited thereto.

In step 105, when the accumulation of the pressure value over time (summation of the AUCs) exceeds a warning threshold, the control device 220 provides a warning signal to operators or medical staffs. Specifically, the medical staffs may set a warning threshold of 120000-360000 (mmHg × min) or 10000-30000 (% × min), but not limited thereto. The warning threshold of the present invention can be modified based on relevant researches on IAH or abdominal compartment syndrome (ACS) in the clinical field, and operators can set a stricter or looser warning threshold based on the object's (T) status (such as age, symptoms, body shape, or gender).

On the other hand, the accumulation of the AUC can only consider the parts that exceeds the critical pressure value (CP). For example, referring to FIG. 8, the critical pressure value (CP) can be set, and only the area beyond the critical pressure value (CP) is calculated and added to the accumulation of the AUC. The accumulation of the AUC can directly reflect the cumulative time that the patient has been exposed to IAH. Accordingly, the accumulation of the AUC for the area beyond the critical pressure value (CP) provides a direct and intuitive assessment of abdominal pressure status.

The present invention discloses the sensing device 210 configured to measure IAP in a continuous, long-term and real-time manner, the control device 220 configured to establish the relationship between IAP and time for the object (T). The AUC of the pressure-time information can be used to evaluate the level of IAP of the object (T). Compared to prior arts, the present invention determines the current IAP of the object (T) directly and immediately; therefore, the sampling rate for measuring IAP is high. The level for patients under IAH can be digitalized and numericized by the calculation of the AUC of the pressure-time information. Accordingly, the present invention reduces the risk of underestimating, overestimating, or misestimating the level of IAH. Moreover, the present invention can avoid waste in human resources or medical resources by calculating the area under the curve of the pressure-time information for IAP.

The previous description of the present invention is provided to enable a person of ordinary skill in the art to make or implement the present invention. Various modifications to the present invention will be apparent to a person skilled in the art, and the general principles defined herein can be applied to other variations without departing from the spirit or scope of the present invention. Therefore, the present invention is not intended to be limited to the examples described herein, but is to be in accord with the widest scope consistent with the principles and novel features of the invention herein.

**Claims**

1. A method (100) for intra-abdominal pressure evaluation and warning, **characterized in that**, comprising:

   arranging a sensing device (210) at an arrangement space inside an abdominal cavity of an object (T);
   measuring a pressure value of the arrangement space by a pressure sensing module (211) of the sensing device (210);
   receiving the pressure value and recording pressure-time information by a control device (220);
   accumulating an area under a curve of the pressure-time information during a measuring duration; and
   while the accumulated area exceeds a warning threshold, outputting a warning signal by the control device (220).

2. The method of claim 1, **characterized in that**: wherein a Y-axis of the pressure-time information is represented as a ratio of the pressure value to a critical pressure value.

3. The method of claim 1, **characterized in that**: wherein the sensing device (210) is arranged at the arrangement space via an esophagus of the object (T).

4. The method of claim 3, **characterized in that**: wherein the arrangement space is selected from any location in a digestive system of the object (T).

5. The method of claim 1, **characterized in that**: wherein the sensing device (210) is communicatively coupled to the control device (220) via a wireless communication.

6. The method of claim 1, **characterized in that**: wherein the pressure sensing module (211) includes a sensor with a piezoelectric, a piezoresistive, a capacitive, an inductive, an electromagnetic piezoelectric, or a thin-film pressure sensing mechanism.

7. A system (200) for intra-abdominal pressure evaluation and warning, **characterized in that**, comprising:

   a sensing device (210) arranged at an arrangement space inside an abdominal cavity of an object (T), the sensing device (210) including a pressure sensing module (211) configured to measure a pressure value of the arrangement space over time; and
   a control device (220) coupled to the sensing device (210), wherein the control device (220) is configured to receive the pressure value and record pressure-time information;
   wherein the control device (220) is further configured to accumulate an area under a curve of the pressure-time information during a measuring duration, and output a warning signal while the area exceeds a warning threshold.

8. The system of claim 7, **characterized in that**: wherein a Y-axis of the pressure-time information is represented as a ratio of the pressure value to a critical pressure value.

9. The system of claim 7, **characterized in that**: wherein the sensing device (210) is arranged at the arrangement space via an esophagus of the object (T).

10. The system of claim 9, **characterized in that**: wherein the arrangement space is selected from any location in a digestive system of the object (T).

11. The system of claim 7, **characterized in that**: wherein the sensing device (210) further includes a wireless transmission module (212) configured to establish a wireless communication; the control device (220) is communicatively coupled to the sensing device (210) via a wireless communication.

12. The system of claim 7, **characterized in that**: wherein the pressure sensing module (211) includes a sensor with a piezoelectric, a piezoresistive, a capacitive, an inductive, an electromagnetic piezoelectric, or a thin-film pressure sensing mechanism.

<u>100</u>

101

arranging a sensing device at an arrangement space
inside an abdominal cavity of an object

102

measuring a pressure value of the arrangement
space by a pressure sensing module of the sensing
device

103

receiving the pressure value and recording
pressure-time information by a control device

104

accumulating an area under a curve of the
pressure-time information during a measuring duration

105

while the accumulated area exceeds a warning threshold,
outputting a warning signal by the control device

FIG. 1

<u>200</u>

FIG. 2

FIG. 3A

210

220

FIG. 3B

200

210

211 → 212

220

FIG. 4

P(mmHg)

Time(min)

FIG. 5

$\delta$ (%)

$$\delta = \frac{P}{CP} \times 100 \ (\%)$$

100

Time(min)

FIG. 6

FIG. 7

FIG. 8

Europäisches
Patentamt
European
Patent Office
Office européen
des brevets

**EUROPEAN SEARCH REPORT**

Application Number

EP 25 15 9445

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/038442 A1 (KCI LICENSING INC [US]) 4 March 2021 (2021-03-04) * figures 1-5 * * paragraphs [0024] - [0035], [0057], [0068] - [0069], [0076] * | 1-12 | INV. A61B5/03 A61B5/07 A61B5/20 A61B5/00 |
| A | MALBRAIN MANU L N G ET AL: "Continuous intra-abdominal pressure: is it ready for prime time?", INTENSIVE CARE MEDICINE, SPRINGER BERLIN HEIDELBERG, BERLIN/HEIDELBERG, vol. 48, no. 10, 4 August 2022 (2022-08-04), pages 1501-1504, XP037932277, ISSN: 0342-4642, DOI: 10.1007/S00134-022-06780-4 [retrieved on 2022-08-04] * page 1503, column 2, lines 1-12 * | 1,7 | |
| A | LIAO CHIEN-HUNG ET AL: "An Ingestible Electronics for Continuous and Real-Time Intraabdominal Pressure Monitoring", JOURNAL OF PERSONALIZED MEDICINE, vol. 11, no. 1, 1 January 2021 (2021-01-01), page 12, XP093176912, ISSN: 2075-4426, DOI: 10.3390/jpm11010012 * figure 1 * * page 4, lines 11-25 * | 3,9 | TECHNICAL FIELDS SEARCHED (IPC) A61B |
| A | US 2012/190938 A1 (ADDINGTON W ROBERT [US] ET AL) 26 July 2012 (2012-07-26) * paragraph [0024] * | 3,9 | |

The present search report has been drawn up for all claims

| Place of search | Date of completion of the search | Examiner |
|---|---|---|
| The Hague | 20 May 2025 | Oancea, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 9445

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2025

| Patent document cited in search report | Publication date | Patent family member(s) | | Publication date |
|---|---|---|---|---|
| WO 2021038442 A1 | 04-03-2021 | NONE | | |
| US 2012190938 A1 | 26-07-2012 | CA | 2824992 A1 | 26-07-2012 |
| | | EP | 2665413 A1 | 27-11-2013 |
| | | US | 2012190938 A1 | 26-07-2012 |
| | | WO | 2012100170 A1 | 26-07-2012 |

EPO FORM P0459

For more details about this annex : see Official Journal of the European Patent Office, No. 12/82